(19) Europäisches Patentamt
European Patent Office
Office européen des brevets

(11) **EP 2 429 606 B1**

(12) **EUROPÄISCHE PATENTSCHRIFT**

(45) Veröffentlichungstag und Bekanntmachung des Hinweises auf die Patenterteilung:
**17.04.2013 Patentblatt 2013/16**

(21) Anmeldenummer: **10722602.9**

(22) Anmeldetag: **12.05.2010**

(51) Int Cl.:
***A61M 1/36*** *(2006.01)*

(86) Internationale Anmeldenummer:
**PCT/EP2010/002945**

(87) Internationale Veröffentlichungsnummer:
**WO 2010/130449 (18.11.2010 Gazette 2010/46)**

(54) **VERFAHREN UND VORRICHTUNG ZUR OPTIMIERUNG EINER EXTRAKORPORALEN BLUTBEHANDLUNG**

METHOD AND APPARATUS FOR OPTIMIZING AN EXTRACORPOREAL BLOOD TREATMENT

PROCÉDÉ ET DISPOSITIF PERMETTANT D'OPTIMISER UN TRAITEMENT SANGUIN EXTRACORPOREL

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**

(30) Priorität: **14.05.2009 DE 102009021255**

(43) Veröffentlichungstag der Anmeldung:
**21.03.2012 Patentblatt 2012/12**

(73) Patentinhaber: **Fresenius Medical Care Deutschland GmbH
61352 Bad Homburg v.d.H. (DE)**

(72) Erfinder:
- **HILGERS, Peter
97453 Schonungen (DE)**
- **TELCHER, Jörg
61350 Bad Homburg (DE)**

(74) Vertreter: **Oppermann, Frank et al
OANDO Oppermann & Oppermann LLP
John-F.-Kennedy-Straße 4
65189 Wiesbaden (DE)**

(56) Entgegenhaltungen:
**EP-A2- 1 159 977**
**EP-A2- 1 614 438**
**JP-A- 9 218 887**
**JP-A- 2000 245 827**
**US-A- 5 319 542**
**US-A- 5 708 798**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 429 606 B1

## Beschreibung

Die Erfindung betrifft ein Verfahren und eine Vorrichtung zur Optimierung einer extrakorporalen Blutbehandlung mit einer extrakorporalen Blutbehandlungsvorrichtung, die einen von einer semipermeablen Membran in eine erste Kammer und eine zweite Kammer unterteilten Dialysator oder Filter aufweist, wobei die erste Kammer Teil eines extrakorporalen Blutkreislaufs und die zweite Kammer des Dialysators oder Filters Teil eines Dialysierflüssigkeitssystems ist. Darüber hinaus betrifft die Erfindung eine extrakorporale Blutbehandlungsvorrichtung mit einer Vorrichtung zur Optimierung der extrakorporalen Blutbehandlung. Die Erfindung betrifft auch ein Computerprogrammprodukt zum Ablauf auf einer Datenverarbeitungseinrichtung zur Durchführung eines Verfahrens zur Optimierung der extrakorporalen Blutbehandlung.

Zur Entfernung von harnpflichtigen Substanzen und zum Flüssigkeitsentzug werden beim chronischen Nierenversagen verschiedene Verfahren zur extrakorporalen Blutbehandlung bzw. -reinigung eingesetzt. Bei der Hämodialyse wird das Blut des Patienten außerhalb des Körpers in einem Dialysator gereinigt. Der Dialysator weist eine Blutkammer und eine Dialysierflüssigkeitskammer auf, die von einer semipermeablen Membran getrennt sind. Während der Behandlung strömt das Blut des Patienten durch die Blutkammer, während Dialysierflüssigkeit durch die Dialysierflüssigkeitskammer strömt, um das Blut des Patienten von den harnpflichtigen Substanzen zu befreien.

Während bei der Hämodialyse (HD) der Transport der kleinmolekularen Substanzen durch die Membran des Dialysators im Wesentlichen durch die Konzentrationsunterschiede (Diffusion) zwischen der Dialysierflüssigkeit und dem Blut bestimmt wird, werden bei der Hämofiltration (HF) im Plasmawasser gelöste Substanzen, insbesondere höhermolekulare Stoffe durch einen hohen Flüssigkeitsstrom (Konvektion) durch die Membran des Dialysators effektiv entfernt. Bei der Hämofiltration fungiert der Dialysator als Filter. Eine Kombination aus beiden Verfahren ist die Hämodiafiltration (HDF).

Es sind verschiedene Dialysatoren oder Filter zur Durchführung der extrakorporalen Blutbehandlungen bekannt. Zu den bekannten Dialysatoren oder Filtern zählen die sogenannten High-Flux- und Low-Flux-Dialysatoren oder Filter. High-Flux-Dialysatoren zeichnen sich durch einen höheren Ultrafiltrationskoeffizienten aus als Low-Flux-Dialysatoren.

Aus der EP 1 614 438 A2 ist eine Vorrichtung zur Konfiguration eines extrakorporalen Blutkreislaufs bekannt, der sich aus verschiedenen Einheiten zusammensetzt, die wiederum verschiedene Komponenten umfassen. Die bekannte Vorrichtung verfügt über eine Datenbank, in der die Einheiten und Komponenten beschreibende Daten gespeichert sind, und eine Eingabeeinheit für die Eingabe einer ausgewählten Einheit aus den zur Verfügung stehenden Einheiten des extrakorporalen Blutkreislaufs. Auf einer Anzeigeeinheit werden nach der getroffenen Auswahl der Einheit die Daten der zu der Einheit gehörenden Komponenten angezeigt.

Für eine extrakorporale Blutbehandlung muss der behandelnde Arzt eine Reihe von Behandlungsparametern vorgeben, die sich einerseits auf den Patienten und andererseits auf die für die Durchführung der Blutbehandlung eingesetzte Blutbehandlungsvorrichtung beziehen. Die vom Arzt vorzugebenden Behandlungsparameter werden daher nachfolgend als patientenspezifische oder maschinenspezifische Behandlungsparameter bezeichnet.

Nachfolgend werden unter patientenspezifischen Behandlungsparametern die Parameter verstanden, die für das Therapieziel und/oder den zu behandelnden Patienten charakteristisch sind. Der behandelnde Arzt gibt zunächst für den Patienten das jeweilige Therapieverfahren vor, wobei beispielsweise zwischen eine Hämodialyse (HD), einer Hämofiltration (HF) und einer Hämodiafiltration (HDF) zu unterscheiden ist. Weiterhin legt der Arzt unter Berücksichtigung der individuellen Kreislaufstabilität des Patienten die Behandlungszeit t fest. Darüber hinaus legt der Arzt als Zielwert für die Behandlung die Dialysedosis kt/V fest. Als Zielwert für eine angemessene Behandlung gilt im Allgemeinen ein Wert für die Dialysedosis kt/V von 1,4. Für Frauen oder besonders leichte Patienten werden in der Literatur auch höhere Zielwerte diskutiert. Ein weiterer patientenspezifischer Behandlungsparameter ist der Fluss des Bluts im extrakorporalen Kreislauf. Der Blutfluss kann zwar vom Arzt während der Blutbehandlung in gewissen Grenzen variiert werden, letztlich ist aber der einstellbare Blutfluss von der Art und den Eigenschaften des Gefäßzugangs des Patienten abhängig.

Unter den maschinenspezifischen Behandlungsparametern werden nachfolgend die Parameter verstanden, die der Arzt selbst unter Berücksichtigung der patientenspezifischen Behandlungsparameter zur Durchführung der extrakorporalen Behandlung frei wählen kann. Zu den maschinenspezifischen Behandlungsparametern zählt insbesondere der Dialysierflüssigkeitsfluss, der bei den bekannten extrakorporalen Blutbehandlungsvorrichtungen innerhalb gewisser Grenzen frei eingestellt werden kann.

Da zur Durchführung der extrakorporalen Blutbehandlung dem behandelnden Arzt unterschiedliche Typen von Dialysatoren oder Filtern zur Verfügung stehen, zu denen zum Beispiel die oben genannten High-Flux- und Low-Flux-Dialysatoren zählen, muss der Arzt vor der Behandlung einen bestimmten Dialysator oder Filter auswählen. Die Auswahl eines bestimmten Dialysatortyps hat aber wiederum Einfluss auf die maschinenspezifischen Behandlungsparameter, die der Arzt zur Erzielung des Therapieziels einstellen muss. Bei unverändertem Blutfluss kann dieselbe Clearance mit einem Dialysator, der sich durch eine größere wirksame Oberfläche auszeichnet, bei einem kleineren Dialysierflüssigkeitsfluss erzielt werden als mit einem Dialysator, der sich durch eine kleinere wirksame Oberfläche auszeichnet.

Der behandelnde Arzt wird die patienten- und maschinenspezifischen Behandlungsparameter so auswählen, dass die Blutbehandlung für den jeweiligen Patienten optimal durchgeführt wird. Wenn dem behandelnden Arzt aber unterschiedliche Typen von Dialysatoren oder Filtern zur Verfügung stehen, können sich unterschiedliche Behandlungsparameter ergeben, mit denen die Blutbehandlung optimal durchgeführt werden kann.

Der Erfindung liegt die Aufgabe zu Grunde, ein Verfahren zur Optimierung einer extrakorporalen Blutbehandlung mit einer extrakorporalen Blutbehandlungsvorrichtung anzugeben, das dem behandelnden Arzt die Auswahl des jeweiligen Dialysators oder Filters aus einer Gruppe von Dialysatoren oder Filtern erleichtert. Darüber hinaus ist eine Aufgabe der Erfindung, eine Vorrichtung zur Optimierung einer extrakorporalen Blutbehandlung zu schaffen, die dem Arzt die Auswahl des Dialysators oder Filters erleichtert. Eine weitere Aufgabe der Erfindung ist, eine extrakorporale Blutbehandlungsvorrichtung mit einer Vorrichtung zur Optimierung der extrakorporalen Blutbehandlung und ein Computerprogrammprodukt zum Ablauf auf einer Datenverarbeitungseinrichtung zur Durchführung des Verfahrens zur Optimierung der extrakorporalen Blutbehandlung bereitzustellen.

Die Lösung dieser Aufgaben erfolgt erfindungsgemäß mit den Merkmalen der Patentansprüche 1, 7, 13 und 14. Bevorzugte Ausführungsformen der Erfindung sind Gegenstand der Unteransprüche.

Das erfindungsgemäße Verfahren und die erfindungsgemäße Vorrichtung zur Optimierung einer extrakorporalen Blutbehandlung beruhen darauf, dass mit einer Recheneinheit für verschiedene Typen von Dialysatoren oder Filter mindestens ein maschinenspezifischer Behandlungsparameter zur Durchführung der Behandlung unter Verwendung des jeweiligen Dialysators oder Filters bestimmt wird, und mit der Recheneinheit die sich aus dem ermittelten maschinenspezifischen Behandlungsparameter unter Verwendung des jeweiligen Dialysators oder Filters ergebenden Kosten bestimmt werden, und auf einer Anzeigeeinheit die ermittelten Kosten für sämtliche Typen von Dialysatoren oder Filtern angezeigt werden. Dadurch werden dem behandelnden Arzt die für die zu treffende Auswahl des Dialysators oder Filters erforderlichen Informationen zur Verfügung gestellt. Der Arzt kann den Dialysator oder Filter dann so auswählen, dass die Kosten der Blutbehandlung möglichst gering sind.

Die erfindungsgemäße Vorrichtung zur Optimierung der extrakorporalen Blutbehandlung kann eine separate Baugruppe bilden, die neben der extrakorporalen Blutbehandlungsvorrichtung betrieben wird. Die erfindungsgemäße Vorrichtung zur Optimierung verfügt über eine Eingabeeinheit zur Eingabe der patienten- und maschinenspezifischen Behandlungsparameter und eine Recheneinheit sowie eine Anzeigeeinheit zur Anzeige der sich für die extrakorporale Blutbehandlung ergebenden Kosten. Es ist aber auch möglich, dass die erfindungsgemäße Vorrichtung Bestandteil einer extrakorporalen Blutbehandlungsvorrichtung ist. Wenn die erfindungsgemäße Vorrichtung Bestandteil einer extrakorporalen Blutbehandlungsvorrichtung ist, kann die erfindungsgemäße Vorrichtung von der Eingabeeinheit, Anzeigeeinheit und/oder Recheneinheit der extrakorporalen Blutbehandlungsvorrichtung Gebrauch machen.

Bei einer bevorzugten Ausführungsform der Erfindung ist der maschinenspezifische Behandlungsparameter, der für die unterschiedlichen Typen von Dialysatoren oder Filtern bestimmt wird, der Dialysierflüssigkeitsfluss, den der behandelnde Arzt für die Durchführung der Blutbehandlung einstellt. Es können aber grundsätzlich auch andere maschinenspezifische Behandlungsparameter als der extrakorporale Blutfluss für sämtliche Typen von Dialysatoren oder Filtern bestimmt werden.

Zur Bestimmung der Kosten der extrakorporalen Blutbehandlung für sämtliche Typen von Dialysatoren oder Filtern sieht das erfindungsgemäße Verfahren und die erfindungsgemäße Vorrichtung vorzugsweise vor, in einer Speichereinheit die Kosten für die unterschiedlichen Typen von Dialysatoren oder Filtern und die Kosten für die Verbrauchsmaterialien und/oder die Energiekosten zu speichern. Die unterschiedlichen Kosten können vorzugsweise auf der Eingabeeinheit eingegeben und dann in die Speichereinheit eingelesen werden, so dass die Kosten jederzeit geändert werden können. Es werden vorzugsweise sowohl die Kosten der Verbrauchsmaterialien als auch die Energiekosten bei der Kostenermittlung berücksichtigt. Es ist aber auch möglich, nur die Kosten der Verbrauchsmaterialien oder der Energiekosten zu berücksichtigen.

Das erfindungsgemäße Verfahren und die erfindungsgemäße Vorrichtung sehen vorzugsweise vor, mit der Recheneinheit die Menge der jeweiligen Verbrauchsmaterialien und/oder die Menge an Energie zu berechnen, die bei der Durchführung der extrakorporalen Blutbehandlung mit dem ermittelten maschinenspezifischen Behandlungsparameter verbraucht wird. Aus den Kosten der Verbrauchsmaterialien und/oder Energiekosten einerseits und der berechneten Menge an Verbrauchsmaterialien und/oder Energie andererseits werden in der Recheneinheit die Kosten der bei der extrakorporalen Blutbehandlung verbrauchten Verbrauchsmaterialien und/oder Energie berechnet. Mit der Recheneinheit werden für sämtliche Typen von Dialysatoren oder Filtern weiterhin die Gesamtkosten jeweils aus der Summe der gespeicherten Kosten des jeweiligen Dialysators oder Filters und den berechneten Kosten der bei der Blutbehandlung verbrauchten Verbrauchsmaterialien und/oder Energie berechnet.

Eine weitere bevorzugte Ausführungsform sieht vor, dass auf der Anzeigeeinheit die unterschiedlichen Beträge des maschinenspezifischen Behandlungsparameters für die unterschiedlichen Dialysatoren oder Filter zusammen mit den sich aus der Verwendung des jeweiligen Dialysators oder Filters und der Einstellung des jeweiligen maschinenspezifischen Behandlungsparameters ergebenden Gesamtkosten der extrakorporalen Blutbehandlung angezeigt werden, so dass der behandelnde Arzt unter Berücksichtigung der Gesamtkosten der Behandlung eine Auswahl zwischen den verschiedenen Dialysatoren oder Filtern treffen kann. Die Gesamtkosten werden vorzugsweise auf der Anzeigeeinheit in diejenigen Kosten aufgegliedert, die sich aus dem Dialysator oder Filter, den Verbrauchsmaterialien und der verbrauchten Energie ergeben.

Im Folgenden wird ein Ausführungsbeispiel der Erfindung unter Bezugnahme auf die Zeichnungen näher erläutert.

Es zeigen:

Fig. 1 die wesentlichen Bestandteile einer extrakorporalen Blutbehandlungsvorrichtung zusammen mit einer Vorrichtung zur Optimierung der Blutbehandlung in stark vereinfachter schematischer Darstellung,

Fig. 2 die Anzeigeeinheit der Vorrichtung zur Optimierung der Blutbehandlung, wobei auf der Anzeigeeinheit der Dialysator oder Filter angezeigt wird, bei dem die Gesamtkosten der Behandlung minimal sind,

Fig. 3 die Anzeigeeinheit der Vorrichtung zur Optimierung der Blutbehandlung, wobei die Kosten für die Blutbehandlung mit einer ersten Blutflussrate für unterschiedliche Typen von Dialysatoren oder Filtern angezeigt werden,

Fig. 4 die Anzeigeeinheit, auf der die Kosten der Blutbehandlung für eine Blutbehandlung mit einer zweiten Blutflussrate, die größer als die erste Blutflussrate ist, für die unterschiedlichen Typen von Dialysatoren oder Filtern angezeigt werden,

Fig. 5 die Anzeigeeinheit bei der Vorgabe anderer Behandlungsparameter, wobei auf der Anzeigeeinheit der Dialysator oder Filter angezeigt wird, mit dem die Kosten der Blutbehandlung minimal sind,

Fig. 6 die Anzeigeeinheit von Fig. 5, wobei die Kosten für die Blutbehandlung mit einer ersten Blutflussrate für unterschiedliche Typen von Dialysatoren oder Filtern angezeigt werden,

Fig. 7 die Anzeigeeinheit von Fig. 5, auf der die Kosten der Blutbehandlung für eine Blutbehandlung mit einer zweiten Blutflussrate, die größer als die erste Blutflussrate ist, für die unterschiedlichen Typen von Dialysatoren oder Filtern angezeigt werden,

Fig. 8 die Anzeigeeinheit, wobei das Therapieziel nicht erreicht wird,

Fig. 9 die Anzeigeeinheit nach einem Optimierungsschritt, für den das nicht erreichte Therapieziel als Zielwert angegeben wird,

Fig. 10 die Anzeigeeinheit, wobei das Therapieziel mit einer Erhöhung der Blutflussrate erreicht wird, und

Fig. 11 die Anzeigeeinheit, wobei das Therapieziel mit einer Erhöhung der Blutflussrate und der Behandlungszeit erreicht wird,

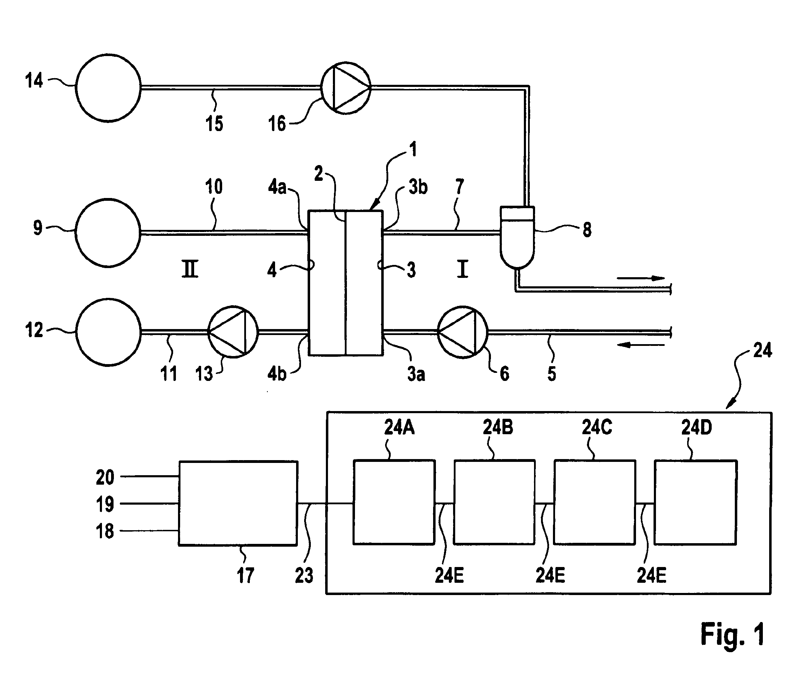

Fig. 1 zeigt in stark vereinfachter schematischer Darstellung die für die Erfindung relevanten Komponenten einer extrakorporalen Blutbehandlungsvorrichtung, die sowohl als Hämodialysevorrichtung und/oder Hämofiltrationsvorrichtung betrieben werden kann. Daher wird die extrakorporale Blutbehandlungsvorrichtung nachfolgend als Hämodiafiltrationsvorrichtung bezeichnet. Die Hämodiafiltrationsvorrichtung weist einen Dialysator oder Filter 1 auf, der durch eine semipermeable Membran 2 in eine Blutkammer 3 und eine Dialysierflüssigkeitskammer 4 unterteilt ist. Der Einlass 3a der Blutkammer ist mit einem Ende einer arteriellen Blutzuführleitung 5 verbunden, in die eine Blutpumpe 6 geschaltet ist, während der Auslass 3b der Blutkammer mit einem Ende einer venösen Blutrückführleitung 7 verbunden ist, in die eine Tropfkammer 8 geschaltet ist. An den anderen Enden der arteriellen und venösen Blutleitung 5, 7 befinden sich die nicht dargestellten arteriellen und venösen Kanülen zum Anschluss an den Patienten. Dieser Teil des Flüssigkeitssystems stellt den extrakorporalen Blutkreislauf I der Hämodiafiltrationsvorrichtung dar.

Das Dialysierflüssigkeitssystem II der Hämodiafiltrationsvorrichtung umfasst eine Einrichtung 9 zur Bereitstellung frischer Dialysierflüssigkeit, die über eine Dialysierflüssigkeitszuführleitung 10 mit dem Einlass 4a der Dialysierflüssigkeitskammer 4 des Dialysators 1 oder Filters verbunden ist. Von dem Auslass 4b der Dialysierflüssigkeitskammer 4 des Dialysators 1 oder Filters geht eine Dialysierflüssigkeitsrückführleitung 11 ab, die zu einem Auslass 12 führt. Zum Fördern der Dialysierflüssigkeit dient eine Dialysierflüssigkeitspumpe 13, die in der Dialysierflüssigkeitsrückführleitung 11 angeordnet ist.

Darüber hinaus verfügt die Hämodiafiltrationsvorrichtung über eine Substituatquelle 14, von der eine Substituatleitung 15, in die eine Substituatpumpe 16 geschaltet ist, zu der venösen Tropfkammer 8 führt. Mit der Substituatpumpe 16 kann dem extrakorporalen Blutkreislauf I eine vorgegebenen Menge an Substitutionsflüssigkeit aus der Substituatquelle 14 zugeführt werden, wenn dem Blutkreislauf über den Dialysator 1 Flüssigkeit entzogen wird.

Die Hämodiafiltrationsvorrichtung umfasst weiterhin eine zentrale Steuer- und Recheneinheit 17, die über Steuerleitungen 18, 19, 20 mit der Blutpumpe 6, der Dialysierflüssigkeitspumpe 13 und der Substituatpumpe 16 verbunden ist. Die zentrale Steuer- und Recheneinheit 17 steuert die Pumpen 6, 13 und 16 derart an, dass sich im extrakorporalen Blutkreislauf I eine bestimmte Blutflussrate $Q_b$ und in dem Dialysierflüssigkeitssystem II eine bestimmte Dialysierflüssigkeitsrate $Q_d$ einstellt. Die Steuer- und Recheneinheit gibt auch eine bestimmte Substituatrate vor und stellt die Ultrafiltrationsrate ein, mit der Flüssigkeit dem Patienten entzogen wird. Die einzelnen Behandlungsparameter, mit denen die extrakorporale Blutbehandlung durchgeführt werden soll, können von dem behandelnden Arzt auf einer nicht dargestellten Eingabeeinheit eingegeben werden.

Nachfolgend wird die erfindungsgemäße Vorrichtung zur Optimierung der Blutbehandlung beschrieben, die eine selbständige Einheit bilden oder Bestandteil der extrakorporalen Blutbehandlungsvorrichtung sein kann. Wenn die Vorrichtung zur Optimierung der Blutbehandlung Bestandteil der Blutbehandlungsvorrichtung ist, kann die Vorrichtung zur Optimierung der Blutbehandlung von den Komponenten Gebrauch machen, die bereits in der Blutbehandlungsvorrichtung vorhanden sind. Beispielsweise kann die Vorrichtung zur Optimierung der Blutbehandlung von der zentralen Rechen- und Speichereinheit 17 Gebrauch machen.

Die Vorrichtung 24 zur Optimierung der Blutbehandlung verfügt bei dem vorliegenden Ausführungsbeispiel über eine eigene Recheneinheit 24A, eine eigene Speichereinheit 24B sowie eine separate Eingabeeinheit 24C und separate Anzeigeeinheit 24D, die untereinander über Datenleitungen 24E miteinander verbunden sind. Mit der zentralen Rechen- und Steuereinheit 17 ist die Vorrichtung 24 über eine weitere Datenleitung 23 verbunden.

Die Rechen- und Speichereinheit 24A, 24B kann Teil einer zentralen Datenverarbeitungsanlage sein, auf der ein Datenverarbeitungsprogramm läuft, wobei die Eingabeeinheit 24C eine Tastatur und die Anzeigeeinheit 24D ein Bildschirm sein kann.

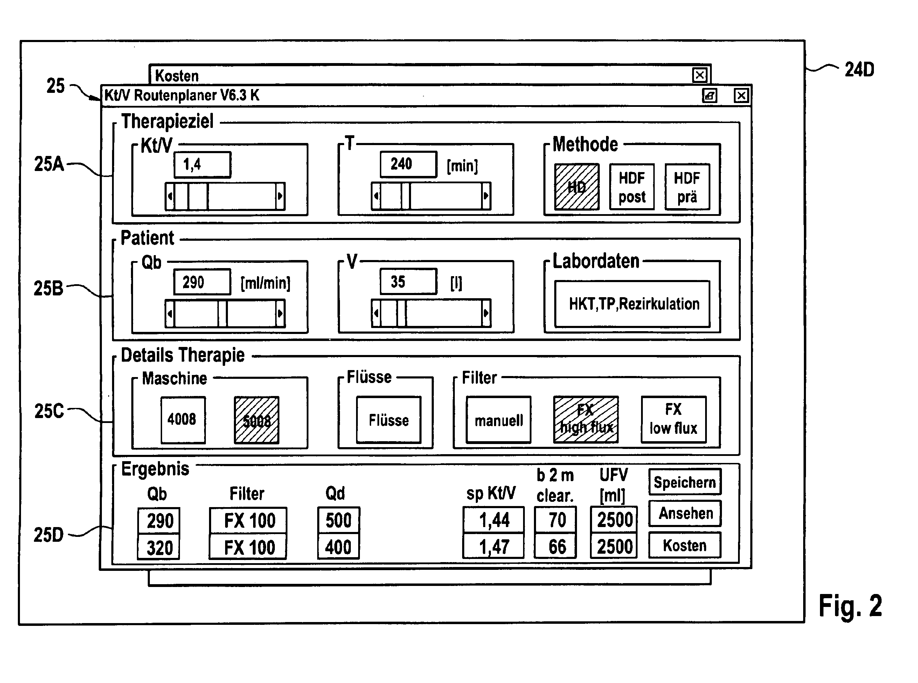

Fig. 2 zeigt den Bildschirm 24D der erfindungsgemäßen Vorrichtung. Auf dem Bildschirm werden die Behandlungsparameter angezeigt, die von dem behandelnden Arzt auf der Eingabeeinheit 24C eingegeben oder von der Recheneinheit 24A berechnet werden. Die Parameter werden mit Hilfe von Masken 25 auf dem Bildschirm dargestellt.

Bei dem vorliegenden Ausführungsbeispiel gibt der behandelnde Arzt zunächst die patientenspezifischen Behandlungsparameter vor, die für das Therapieziel und den zu behandelnden Patienten charakteristisch sind. Die für das Therapieziel charakteristischen Behandlungsparameter werden auf dem Bildschirm in der Eingabemaske 25 in der ersten Zeile 25A dargestellt, während die für den Patienten charakteristischen Parameter in der zweiten Zeile 25B dargestellt werden. Das Therapieziel gibt der Arzt mit den Behandlungsparametern der Dialysedosis Kt/V, der Behandlungsdauer T und der Behandlungsmethode Hämodialyse HD, Hämodiafiltration HDF mit Postdilution $HDFp_{ost}$ bzw. Prädilution $HDF_{prä}$ vor. Für den Patienten gibt der Arzt einen bestimmten Blutfluss $Q_b$, ein bestimmtes Harnstoffverteilungsvolumen V sowie verschiedene weitere Labordaten vor, zu denen beispielsweise der Hämatokrit HKT zählen können. Für die Dialysedosis Kt/V kann eine tolerierte Abweichung von dem Zielwert vorgegeben werden.

Im vorliegenden Ausführungsbeispiel werden für die Dialysdosis Kt/V 1,4, die Behandlungszeit T 240 min, die Behandlungsmethode Hämodialyse HD, den Blutfluss $Q_b$ 290 ml/min und das Harnstoffverteilungsvolumen V 35 1 vorgegeben. Ferner wird als tolerierte Abweichung von dem Zielwert für die Dialysedosis Kt/V 2 % vorgegeben.

Darüber hinaus kann der Arzt vorgeben, ob vorzugsweise High-Flux-Dialysatoren oder Filter oder Low-Flux-Dialysatoren oder Filter verwendet werden sollen. Ferner kann der Arzt den Typ der Blutbehandlungsvorrichtung vorgeben. Dies wird in Spalte 25C der Maske 25 eingestellt. Hier hat der Arzt High-Flux-Dialysatoren oder Filter vorgegeben und eine Dialysemaschine des Typs 5008 ausgewählt, was durch farbliche Unterlegung der einzelnen Felder angezeigt werden kann. Um den Arzt zu animieren, über eine Erhöhung des Blutflusses nachzudenken, kann ein weiterer Wert für den Blutfluss eingegeben werden, der größer als der erste Wert ist. Die spätere Berechnung erfolgt dann für den niedrigeren und den höheren Blutfluss. Hier hat der Arzt für den erhöhten Blutfluss $Q_b$ 320 ml/min eingegeben. Beide Blutflüsse werden in der Spalte 25D angezeigt.

Da eine Erhöhung des Dialysierflüssigkeitsflusses über einen vorgegebenen oberen Grenzwert hinaus in der Praxis zu keiner wesentlichen Steigerung der Clearance K führt, kann für den Dialysierflüssigkeitsfluss ein oberer Grenzwert vorgegeben werden. Bei Patienten mit einem kleinem Verteilungsvolumen V kann es vorkommen, dass der Zielwert für die Dialysedosis Kt/V schon mit einem Dialysierflüssigkeitsfluss erreicht wird, der kleiner als der Blutfluss ist. In solchen Fällen kann ein Dialysierflüssigkeitsfluss vorgegeben werden, der gleich dem Blutfluss ist. Auch kann ein vorgegebener unterer Grenzwert für den Dialysierflüssigkeitsfluss vorgegeben werden. Die Vorgabe oberer bzw. untere Grenzwerte für den Dialysierflüssigkeitsfluss oder Blutfluss ist aber nur eine Option.

Die erfindungsgemäße Vorrichtung 24 zur Optimierung der Blutbehandlung bestimmt nunmehr sowohl für den vorgegebenen niedrigeren Blutfluss als auch den höheren Blutfluss von 290 bzw. 320 ml/min den Typ des Dialysators oder Filters, mit dem bei einem bestimmten Dialysierflüssigkeitsfluss $Q_d$ die Kosten der Dialysebehandlung minimal sind. Bei dem vorliegenden Ausführungsbeispiel schlägt die Vorrichtung zur Optimierung der Blutbehandlung einen Filter des Typs FX 100 bei einem Dialysierflüssigkeitsfluss $Q_d$ von 500 ml/min vor, wenn der Blutfluss $Q_b$ 290 ml/min ist. Wenn der Blutfluss $Q_b$ 320 ml/min ist, schlägt die Vorrichtung ebenfalls einen Filter des Typs FX 100 vor, wobei allerdings der Dialysierflüssigkeitsfluss $Q_d$ 400 ml/min ist. Nachfolgend wird im Einzelnen beschrieben, wie die Auswertung der Daten erfolgt.

In der Speichereinheit 24B sind verschiedenen Dialysatoren oder Filtern aus einer Gruppe von Dialysatoren oder Filtern jeweils ein bestimmter Blutflussbereich $Q_{b1}$ bis $Q_{b2}$ zugeordnet. Die Gruppe von Dialysatoren oder Filtern umfasst zwei Arten von Filtern, zu denen die High-Flux-Filter und die Low-Flux-Filter zählen. Die High-Flux-Filter beispielsweise umfassen die Typenreihe FX 50, FX 60, FX80 und FX 100.

Zunächst werden aus der vom Arzt gewählten Art des Dialysators oder Filters, beispielsweise eines High-Flux-Filters, und dem gewünschten Blutfluss $Q_b$ diejenigen Filter aus der jeweiligen Typenreihe ausgewählt, die für den gewünschten Blutfluss $Q_b$ grundsätzlich geeignet sind.

Beispiel 1: Ausgewählt FX High-Flux, $Q_b$ = 220 ml/min : FX 50 bis FX 100 sind möglich.

Beispiel 2: Ausgewählt FX High-Flux, $Q_b$ = 370 ml/min : FX 60 bis FX 100 sind möglich.

Die Recheneinheit 24A berechnet aus dem vorgegebenen Wert für die Dialysedosis KT/V, beispielsweise 1,40, der eingegebenen Behandlungszeit T und dem eingegebenen Harnstoffverteilungsvolumen V zunächst einen Zielwert für die Clearance K. Bei der Berechnung des Zielwerts für die Clearance K wird bei dem vorgegebenen Zielwert für die Dialysedosis KT/V die vorgegebene Toleranz, beispielsweise 2 % berücksichtigt, so dass bei der späteren Berechnung der Kosten der Behandlung ein Wert für die Dialysedosis KT/V innerhalb der vorgegebenen Toleranz akzeptiert werden kann. Dadurch lassen sich in einzelnen Fällen Kosten sparen, ohne dass dies in der Praxis nachweisbare Auswirkungen auf den Erfolg der Behandlung hat.

Wenn der Zielwert für die Clearance K bestimmt ist, berechnet die Recheneinheit 24A unter Berücksichtigung des vorgegebenen Behandlungsverfahrens, beispielsweise HD, $HDF_{post}$, $HDF_{prä}$, des vorgegebenen Blutflusses $Q_b$ sowie weiteren Parametern, zu denen beispielsweise der Hämatokrit HKT zählt, für alle Dialysatoren oder Filter, die zuvor bestimmt worden sind, den für die Behandlung jeweils benötigten Dialysierflüssigkeitsfluss $Q_d$. Dabei berechnet die Recheneinheit aus der zuvor bestimmten Clearance K sowie dem vorgegebenen Blutfluss $Q_b$ den Dialysierflüssigkeitsfluss $Q_d$, der erforderlich ist, um die gewünschte Clearance K zu erzielen. Die Berechnung des erforderlichen Dialysierflüssigkeitsflusses $Q_d$ erfolgt für eine HD-Behandlung mit dem Koeffizienten k0A auf der Grundlage der folgenden Gleichung:

$$K = Q_b Q_d \frac{1-\exp\left(-k0A\frac{Q_d-Q_b}{Q_d Q_b}\right)}{Q_d - Q_b\exp\left(-k0A\frac{Q_d-Q_b}{Q_d Q_b}\right)}$$

Für andere Behandlungsarten, wie beispielsweise HDF können andere Gleichungen zur Berechnung von K zur Anwendung kommen. Der Koeffizient k0A ist eine für den jeweiligen Dialysator oder Filter charakteristische Größe, die im Wesentlichen von der aktiven Oberfläche der semipermeablen Membran des Dialysators oder Filters und deren Diffusionswiderstand abhängig ist.

In der Speichereinheit 24B sind für sämtliche Dialysatoren oder Filter die jeweiligen Koeffizienten k0A gespeichert, die von der Recheneinheit 24A zur Berechnung der Dialysierflüssigkeitsrate $Q_d$ aus dem Speicher ausgelesen werden. Das oben angegebene Verfahren zur Bestimmung der Dialysierflüssigkeitsrate ist in der WO 2007/140993 A1 im Einzelnen beschrieben, auf deren Offenbarung ausdrücklich Bezug genommen wird. Für die Erfindung ist aber letztlich unerheblich, nach welchem Verfahren die Dialysierflüssigkeitsrate bestimmt wird.

In der Speichereinheit 24B sind weiterhin die Kosten von verschiedenen Verbrauchsmaterialien und die Energiekosten gespeichert. Alle notwendigen Kosten (Verkaufspreise) können durch entsprechende Menüs eingegeben werden. Bei dem vorliegenden Ausführungsbeispiel umfassen die Kosten der Verbrauchsmaterialien die Kosten für Permeat, Säure und Bicarbonat und die Energiekosten umfassen die Kosten für den elektrischen Strom. In der Speichereinheit 24B sind auch die Kosten (Verkaufspreise) der einzelnen Dialysatoren oder Filter gespeichert.

Für sämtliche Dialysatoren oder Filter, die für die Blutbehandlung vorgeschlagen werden, berechnet die Recheneinheit 24B unter Berücksichtigung der jeweiligen Dialysierflüssigkeitsraten $Q_d$ sowie der vorgegebenen Blutflussraten $Q_b$ die Menge der jeweiligen Verbrauchsmaterialien (Permeat, Säure, Bicarbonat) sowie die Menge an Energie (elektrischer Strom), die bei der Blutbehandlung verbraucht wird.

Aus der berechneten Menge der Verbrauchsmaterialien und den gespeicherten Kosten der Verbrauchsmaterialien sowie der verbrauchten Energie und den Energiekosten berechnet die Recheneinheit 24A nunmehr die Kosten für sämtliche Verbrauchsmaterialien und die Kosten für die mit der Behandlung verbrauchten Energie. Schließlich berechnet die Recheneinheit die Gesamtkosten für sämtliche Typen von Dialysatoren oder Filtern jeweils aus der Summe der zuvor bestimmten Kosten für die bei der Behandlung verbrauchten Verbrauchsmaterialien und der Kosten für die verbrauchte Energie sowie der Kosten der Dialysatoren oder Filter.

Die endgültige Auswahl des Dialysators oder Filters erfolgt dann unter der Prämisse, dass die Gesamtkosten minimal sind.

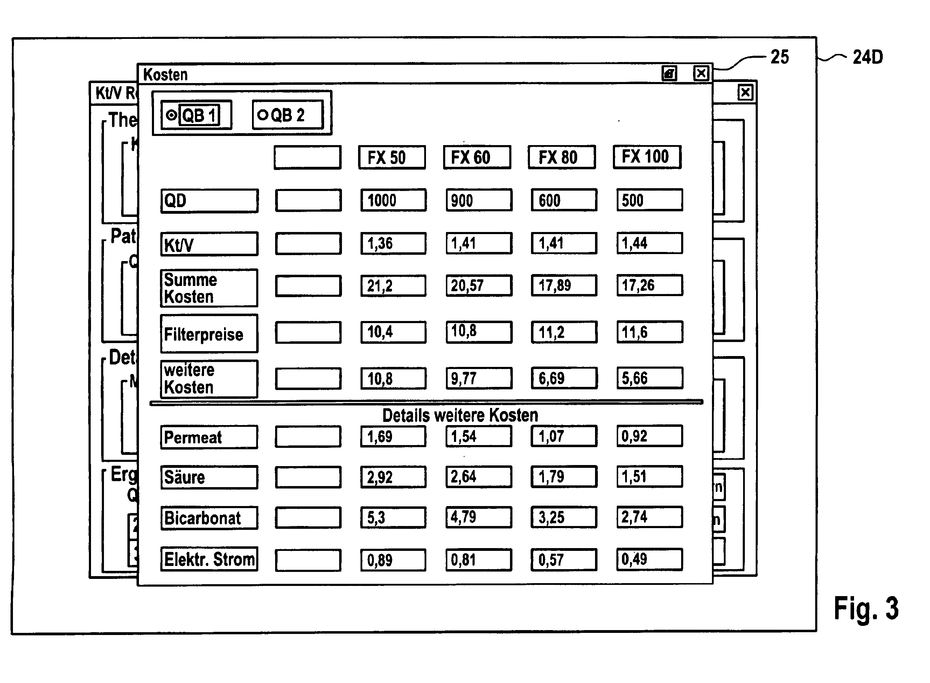

Der Arzt kann über den Menüpunkt "Kosten" ein Untermenü aufrufen, in dem die einzelnen Kosten für die vorgeschlagenen Dialysatoren oder Filter dargestellt werden. Fig. 3 zeigt das Untermenü für das vorliegende Ausführungsbeispiel, bei dem der Blutfluss $Q_b$ von 290 ml/min ($Q_b$ 1)vorgegeben ist (Fig. 2).

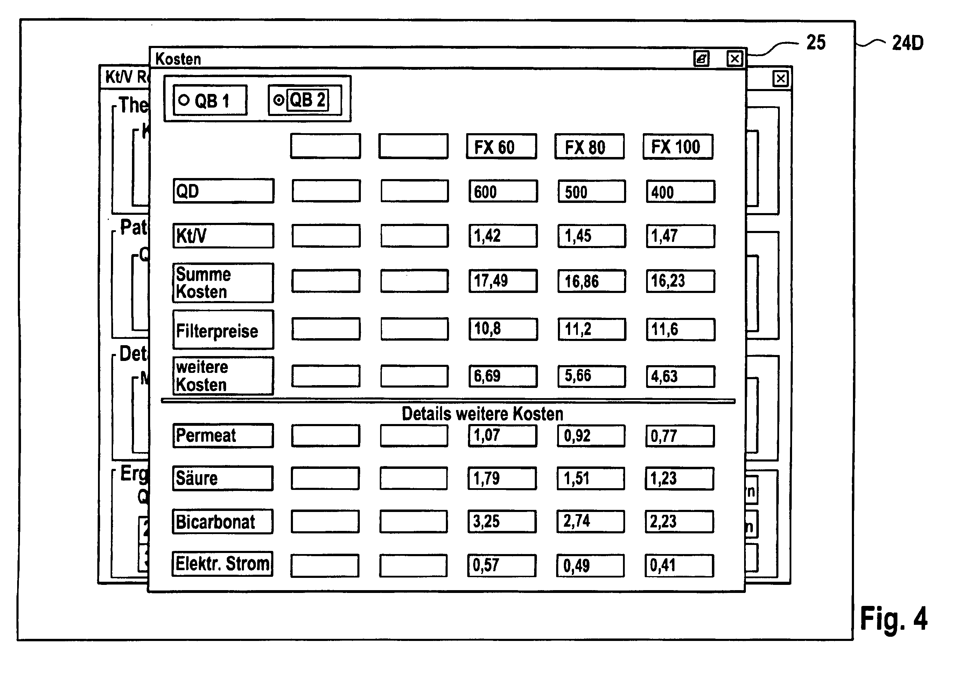

Bei der Kostendarstellung werden stets alle Dialysatoren oder Filter spaltenweise angezeigt, die für den vorgesehenen Blutfluss geeignet sind. Dies sind bei dem vorliegenden Ausführungsbeispiel, bei dem ein Blutfluss von 290 ml/min vorgegeben ist, die High-Flux-Dialysatoren FX 50 bis FX 100. Dabei werden diejenigen Dialysatoren oder Filter durch farbliche Unterlegung der Menüpunkte gekennzeichnet, mit denen der Zielwert für die Dialysedosis Kt/V nicht erreicht werden kann, mit denen der Zielwert für die Dialysedosis nur mit einem relativ hohen Dialysierflüssigkeitsfluss oder mit einem relativ niedrigen Dialysierflüssigkeitsfluss erreicht werden kann. Bei dem vorliegenden Ausführungsbeispiel ist der Filter FX 50 mit der Farbe rot gekennzeichnet, da der Zielwert für die Dialysedosis von 1,40 mit diesem Filter nicht erreicht werden kann. Der Filter FX 60 ist mit der Farbe gelb gekennzeichnet, da mit diesem Filter der Zielwert für die Dialysedosis nur mit einem relativ hohen Dialysierflüssigkeitsfluss, der mehr als das 1,5-fache des Blutflusses beträgt, erreicht werden kann. Die Filter FX 80 und FX 100 hingegen sind mit der Farbe grün gekennzeichnet, da mit diesen Filtern bei einem relativ niedrigen Dialysierflüssigkeitsfluss der Zielwert für die Dialysedosis erreicht wird. Es zeigt sich bei dem vorliegenden Ausführungsbeispiel, dass die Summe der Kosten, die sich aus den Filterpreisen und den weiteren Kosten zusammensetzen, die Kosten für Permeat, Säure, Bicarbonat und elektrischen Strom umfassen, bei dem Filter FX 100 minimal sind. Die Recheneinheit 24A wählt nach einem Vergleich der unterschiedlichen Gesamtkosten daher den Dialysator FX 100 aus, bei dem die Gesamtkosten minimal sind. Das Ergebnis wird in dem Hauptmenü angezeigt (Fig. 2: $Q_b$ 290, Filter FX 100 $Q_d$ 500). Fig. 4 zeigt das Untermenü für den alternativen höheren Blutfluss von 320 ml/min ($Q_b$ 2). Bei dem erhöhten Blutfluss zeigt sich, dass der Filter FX 50 nicht mehr in Frage kommt. Dieser Filter wird daher nicht mehr in der Maske angezeigt. Der Zielwert für die Dialysedosis Kt/V kann bei dem erhöhten Blutfluss schon mit einem Dialysierflüssigkeitsfluss von 400 ml/min erreicht werden, wenn der Filter FX 100 verwendet wird. Bei diesem Filter sind auch die Gesamtkosten der Behandlung am geringsten. Folglich schlägt die Recheneinheit für die Behandlung auch bei dem höheren Blutfluss von 320 ml/min wieder den Filter FX 100 vor (Fig. 2: $Q_b$ 320, Filter FX 100 $Q_d$ 400). Dem Arzt bleibt nunmehr überlassen, ob er die Behandlung mit dem Filter FX 100 bei dem niedrigeren oder höheren Blutfluss mit dem höheren bzw. niedrigeren Dialysierflüssigkeitsfluss durchführt. Das Ausführungsbeispiel zeigt somit, dass die erfindungsgemäße Vorrichtung dem Arzt besonders deutlich macht, welche Auswirkungen Änderungen des Blutflusses oder der Behandlungszeit haben.

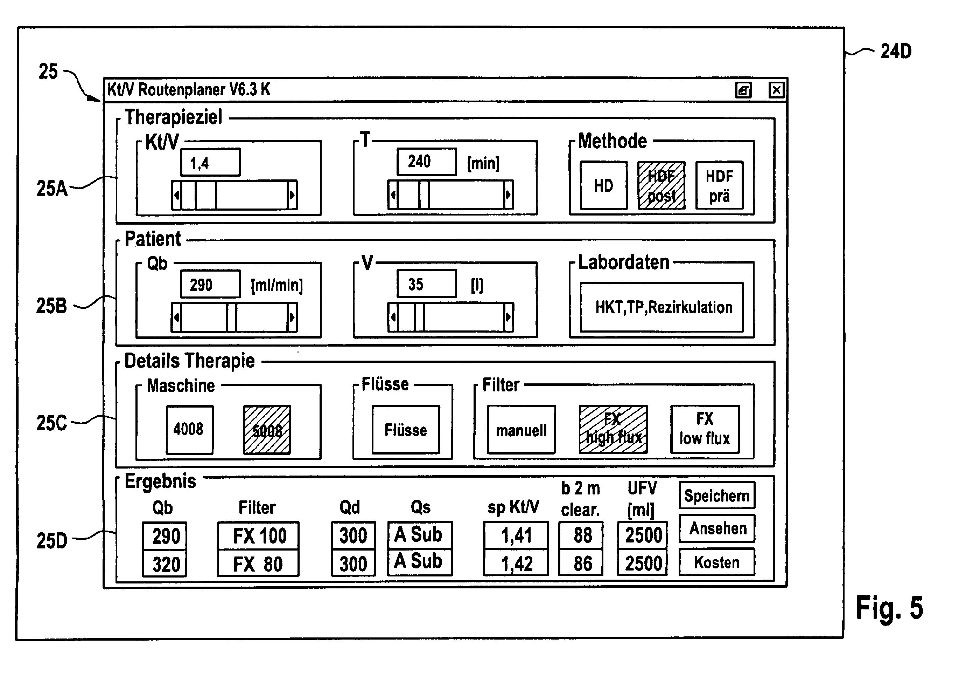

Die Figuren 5 bis 7 zeigen das Hauptmenü und die beiden Untermenüs, die auf der Anzeigeeinheit 24D der erfindungsgemäßen Vorrichtung zur Optimierung der Blutbehandlung dargestellt werden, wenn nicht eine HD-Behandlung, sondern eine HDF-Behandlung (Hämodiafiltration) mit Postdilution ($HDF_{post}$) vorgegeben wird. Ansonsten sind die Behandlungsparameter mit den Parametern identisch, mit denen die Behandlung bei dem ersten Ausführungsbeispiel durchgeführt wird.

In dem Hauptmenü (Fig. 5) wird für den Blutfluss $Q_b$ 290 ml/min der Filter FX 100 bei einem Dialysierflüssigkeitsfluss von 300 ml/min und der Filter FX 80 für einen Blutfluss $Q_b$ von 320 ml/min bei einem Dialysierflüssigkeitsfluss von ebenfalls 300 ml/min vorgeschlagen, um die Kosten der Behandlung möglichst gering zu halten, ohne den Zielwert für die Dialysedosis aufzugeben.

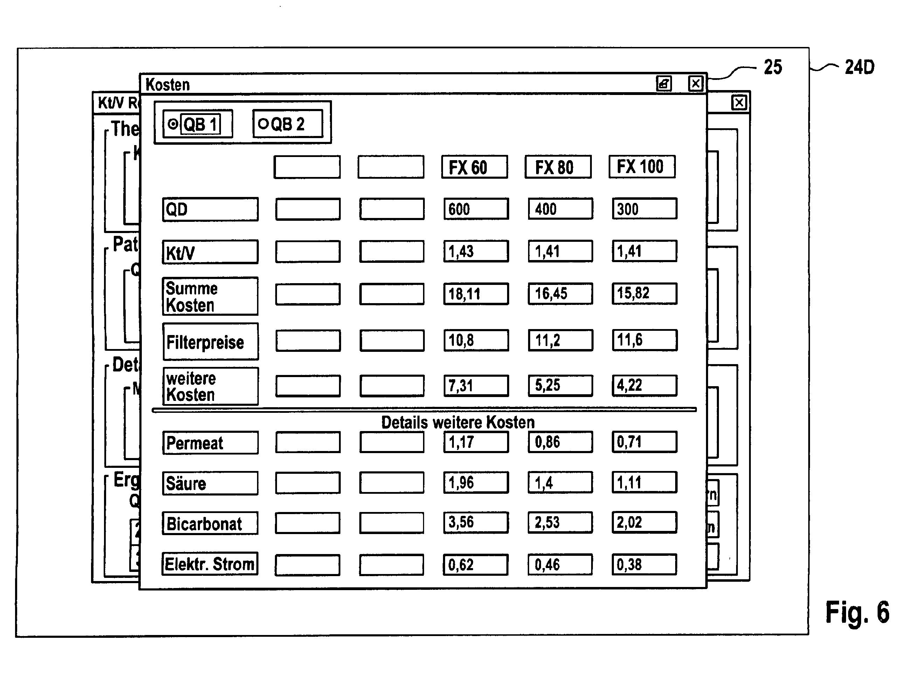

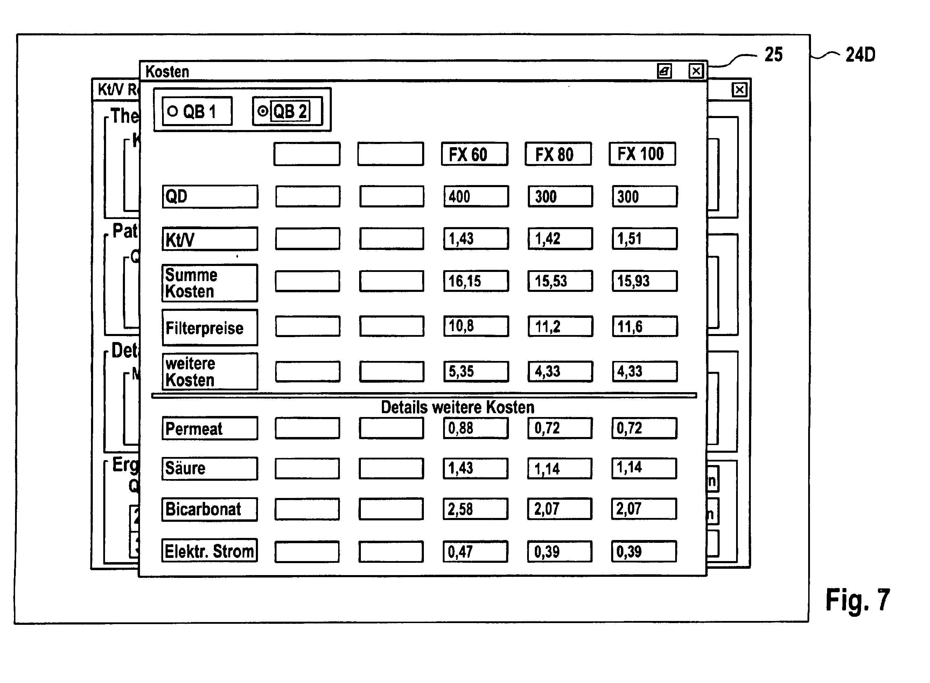

Das Untermenü für den Blutfluss $Q_b$ von 290 ml/min (Fig. 6) zeigt, dass eine Behandlung grundsätzlich mit den Filtern FX 60 bis FX 100 möglich ist. Für diese Filter berechnet die Recheneinheit einen Dialysierflüssigkeitsfluss, der zwischen 600 und 300 ml/min liegt. Mit sämtlichen Filtern kann der Zielwert der Dialysedosis von 1,40 erreicht werden. Die Gesamtkosten der Behandlung sind jedoch mit dem Filter FX 100 am geringsten. Auch ist der Dialysierflüssigkeitsfluss mit dem Filter FX 100 am geringsten. Eine Erhöhung des Blutflusses auf 320 ml/min führt dazu, dass die Gesamtkosten der Behandlung mit dem Filter FX 80 am geringsten sind (Fig. 7). Mit dem Filter FX 80 ist auch der Dialysierflüssigkeitsfluss am geringsten. Dieser entspricht dem Dialysierflüssigkeitsfluss mit dem Filter FX 100 bei dem niedrigeren Blutfluss.

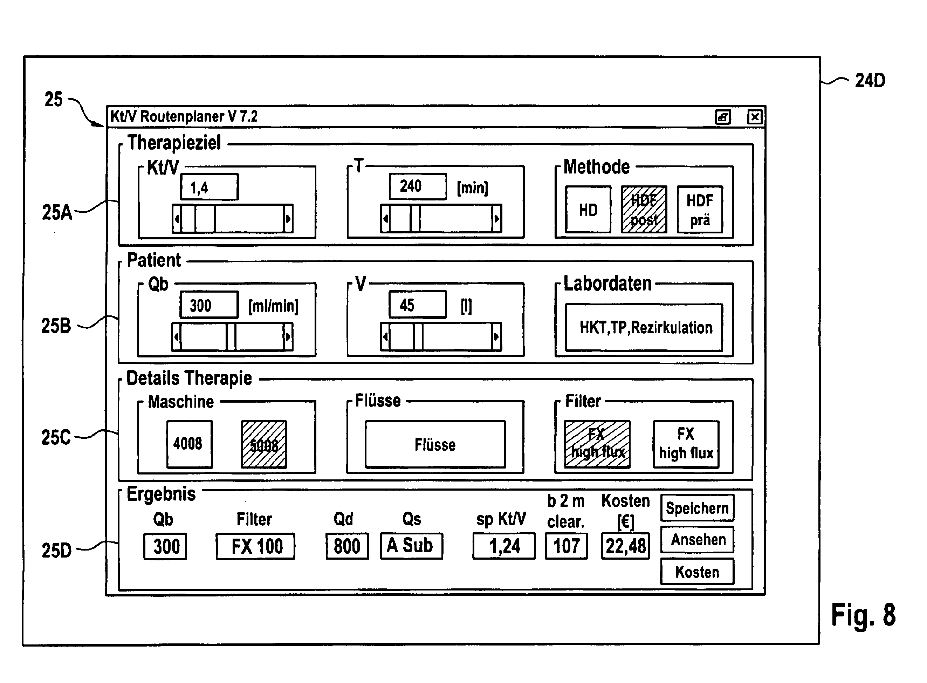

Unter Bezugnahme auf die Figuren 8 bis 11 wird der Fall beschrieben, dass auch innerhalb der vorgegebenen Toleranz für die Dialysedosis KT/V das Therapieziel nicht erreicht werden kann.

Fig. 8 zeigt den Bildschirm 24D, auf dem der vorgegebene Wert für die Dialysedosis KT/V angezeigt wird. Der Arzt hat eine Dialysedosis KT/V von 1,4 vorgegeben. Wenn das Therapieziel beispielsweise bei geringem Blutfluss $Q_b$ oder großem Verteilungsvolumen V nicht erreicht wird, erhält der Arzt einen Hinweis. Bei dem vorliegenden Ausführungsbeispiel wird der erzielbare Wert für die Dialysedosis KT/V von 1,24, der unter dem vorgegebenen Wert von 1,4 liegt, d.h. auch außerhalb der vorgegebenen Toleranz von 2 % liegt, auf einem rotem Hintergrund dargestellt. Damit wird signalisiert, dass das Therapieziel nicht erreicht wird.

In diesem Fall müsste immer der größte Dialysator und der höchste Dialysierflüssigkeitsfluss $Q_d$ gewählt werden, um dem Zielwert möglichst nahe zu kommen. Ein effektiver Einsatz der Mittel ist aber damit nicht gegeben.

Die Recheneinheit 24A führt in diesem Fall automatisch einen weiteren Optimierungsschritt durch, bei dem der in dem ersten Optimierungsschritt erreichte Wert für die Dialysedosis KT/V, beispielsweise 1,24, als neuer Zielwert vorgegeben wird. Auch für den neuen Zielwert berücksichtigt die Recheneinheit 24A wieder eine vorgegebene Toleranz, beispielsweise 2 %.

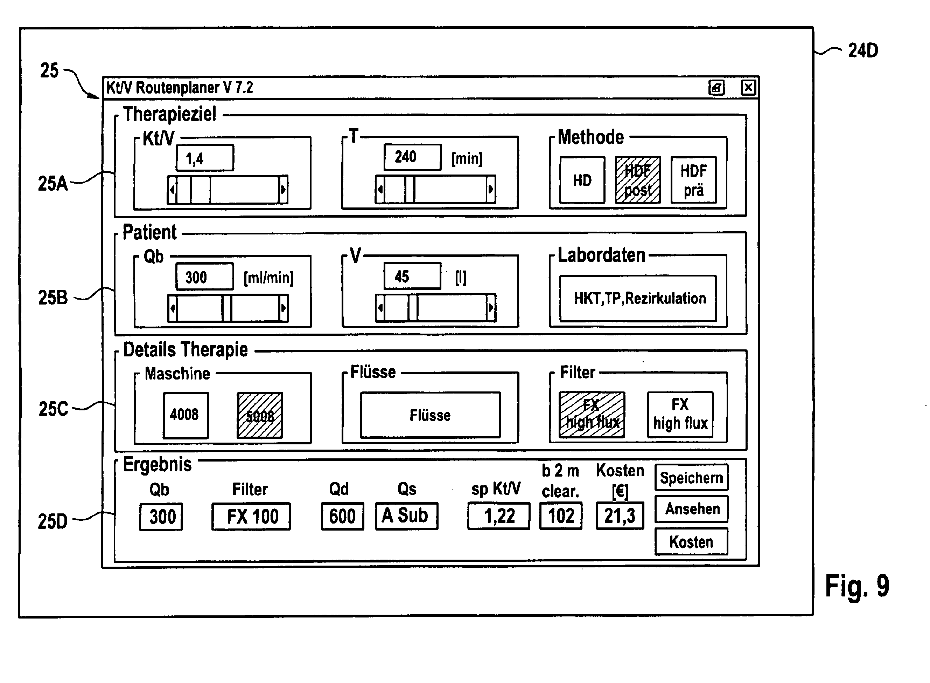

Fig. 9 zeigt den Bildschirm 24D, auf dem das Ergebnis der Berechnung in dem zweiten Optimierungsschritt mit einem Zielwert für die Dialysedosis KT/V von 1,24 dargestellt ist. Es zeigt sich, dass sich eine Dialysedosis KT/V von 1,22 ergibt, die zwar wieder außerhalb der vorgegebenen Toleranz liegt, aber nur geringfügig kleiner als der vorgegebene Zielwert von 1,24 ist Da der neu berechnete Wert für die Dialysedosis KT/V wieder außerhalb der Toleranz liegt, wird der Wert wieder auf rotem Hintergrund dargestellt. Die Recheneinheit 24A berechnet die Kosten, die sich bei den vorgegebenen Parametern ergeben, die auf dem Bildschirm 24D angezeigt werden. Die Kosten belaufen sich bei dem vorliegenden Ausführungsbeispiel auf EUR 21,30.

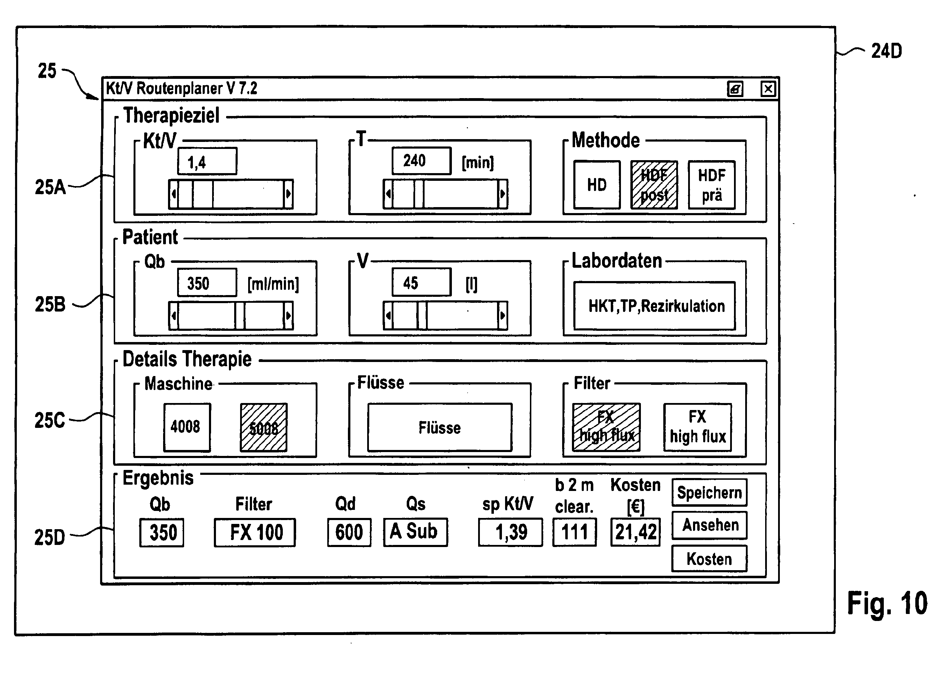

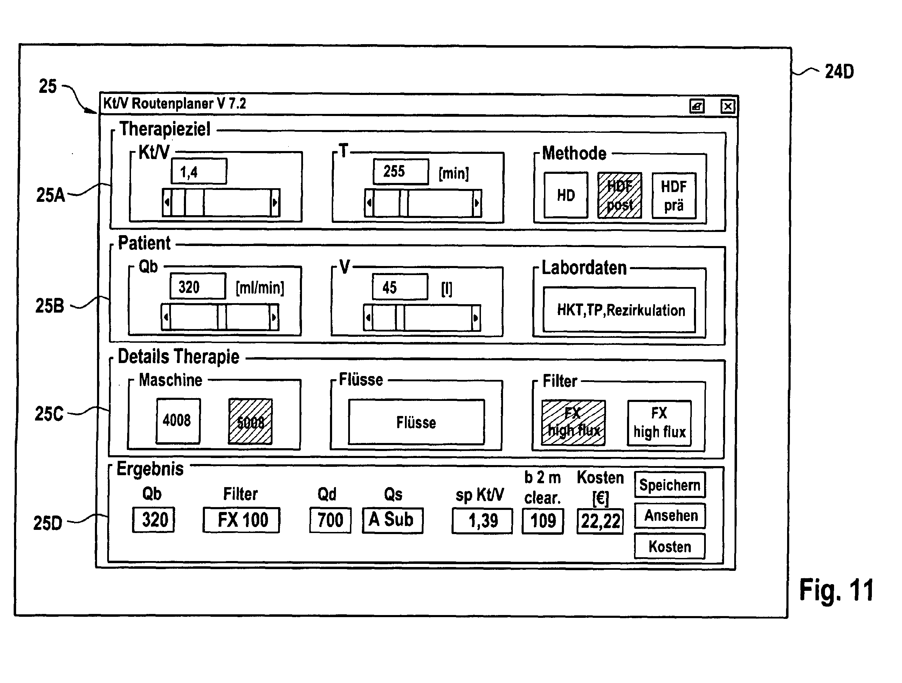

Die Figuren 10 und 11 zeigen, dass eine Erhöhung der Dialysedosis KT/V auf 1,4 mit einer Erhöhung des Blutflusses $Q_b$ und/oder der Behandlungszeit T erreicht werden kann. Beispielsweise kann eine Dialysedosis KT/V von 1,39, die innerhalb der Toleranz liegt, dadurch erreicht werden, dass der Blutfluss $Q_b$ bei gleicher Behandlungszeit T von 240 min von 300 auf 350 ml/min erhöht wird (Fig. 10). Eine Dialysedosis KT/V von 1,39 kann aber auch mit einem Blutfluss $Q_b$ von 320 ml/min bei einer Behandlungszeit T von 255 erreicht werden (Fig. 11). Die Recheneinheit 24A berechnet für die unterschiedlichen Vorgaben die Kosten, die sich auf EUR 21,42 (Fig. 10) bzw. EUR 22,22 (Fig. 11) belaufen, so dass der Arzt eine Entscheidung treffen kann, mit welchen Parametern die Behandlung durchgeführt werden soll.

## Patentansprüche

1. Verfahren zur Auswahl eines Dialysators oder Filters unter Kostengesichtspunkten für eine extrakorporale Blutbehandlung mit einer extrakorporalen Blutbehandlungsvorrichtung, die einen von einer semipermeablen Membran in eine erste Kammer und eine zweite Kammer unterteilten Dialysator oder Filter aufweist, wobei die erste Kammer Teil eines extrakorporalen Blutkreislaufs und die zweite Kammer des Dialysators oder Filters Teil eines Dialysierflüssigkeitssystems ist, **gekennzeichnet durch** die folgenden Verfahrensschritte:

   Eingabe von einem oder mehreren patientenspezifischen Behandlungsparametern auf einer Eingabeeinheit, die für das Therapieziel und/oder den zu behandelnden Patienten charakteristisch sind,
   Bestimmen eines maschinenspezifischen Behandlungsparameters aus dem einen oder aus den mehreren patientenspezifischen Behandlungsparametern zur Durchführung der Behandlung unter Verwendung von jeweils einem der Dialysatoren oder Filter aus einer Gruppe von unterschiedlichen Typen von Dialysatoren oder Filtern für sämtliche Typen von Dialysatoren oder Filtern mit einer Recheneinheit,
   Bestimmen der sich aus dem ermittelten maschinenspezifischen Behandlungsparameter unter Verwendung des jeweiligen Dialysators oder Filters ergebenden Kosten der extrakorporalen Blutbehandlung für sämtliche Typen von Dialysatoren oder Filter mit der Recheneinheit,
   Anzeigen der sich aus dem maschinenspezifischen Behandlungsparameter unter Verwendung des jeweiligen Dialysators oder Filters ergebenden Kosten auf einer Anzeigeeinheit für sämtliche Typen von Dialysatoren oder Filtern.

2. Verfahren nach Anspruch 1, **dadurch gekennzeichnet, dass** der Schritt des Bestimmens der Kosten der extrakorporalen Blutbehandlung folgende Schritte umfasst:

   Speichern der Kosten der unterschiedlichen Typen von Dialysatoren oder Filter in einer Speichereinheit,
   Speichern der Kosten von Verbrauchsmaterialien und/oder der Energiekosten in der Speichereinheit,
   Berechnen der Menge der jeweiligen Verbrauchsmaterialien und/oder der Menge an Energie in der Recheinheit, die bei der Durchführung der extrakorporalen Blutbehandlung mit dem ermittelten maschinenspezifischen Behandlungsparameter verbraucht wird,
   Berechnen der Kosten der bei der extrakorporalen Blutbehandlung verbrauchten Verbrauchsmaterialien und/oder Energie aus den Kosten der Verbrauchsmaterialien und/oder Energiekosten einerseits und der berechneten Menge an Verbrauchsmaterialien und/oder Energie andererseits,
   wobei mit der Recheneinheit die Gesamtkosten für sämtliche Typen von Dialysatoren oder Filtern jeweils aus der Summe der gespeicherten Kosten des jeweiligen Dialysators oder Filters und den berechneten Kosten der bei der extrakorporalen Blutbehandlung verbrauchten Verbrauchsmaterialien und/oder Energie berechnet werden.

**3.** Verfahren nach Anspruch 1 oder 2, **dadurch gekennzeichnet, dass** die unterschiedlichen Beträge des maschinenspezifischen Behandlungsparameters für die unterschiedlichen Dialysatoren oder Filter sowie die sich aus der Verwendung des jeweiligen Dialysators oder Filters und der Einstellung des jeweiligen maschinenspezifischen Behandlungsparameters ergebenden Gesamtkosten der extrakorporalen Blutbehandlung auf der Anzeigeeinheit angezeigt werden.

**4.** Verfahren nach einem der Ansprüche 1 bis 3, **dadurch gekennzeichnet, dass** der zu bestimmende maschinenspezifische Behandlungsparameter der Dialysierflüssigkeitsfluss ist, mit dem Dialysierflüssigkeit durch die zweite Kammer des Dialysators oder Filters strömt.

**5.** Verfahren nach einem der Ansprüche 1 bis 4, **dadurch gekennzeichnet, dass** ein patientenspezifischer Parameter ein Parameter aus der Gruppe von Parametern ist, die das Blutverteilungsvolumen V, die Dauer T der extrakorporalen Blutbehandlung, die Dialysedosis, die Clearance K oder den Blutfluss $Q_b$ im extrakorporalen Blutkreislauf I umfasst.

**6.** Verfahren nach einem der Ansprüche 1 bis 5, **dadurch gekennzeichnet, dass** mit der Recheneinheit derjenige Dialysator oder Filter ausgewählt wird, bei dem die sich für die Behandlung ergebenden Kosten am geringsten sind, und dass auf der Anzeigeeinheit dieser Dialysator oder Filter angezeigt wird.

**7.** Vorrichtung zur Auswahl eines Dialysators oder Filters unter Kostengesichtspunkten für eine extrakorporale Blutbehandlung mit einer extrakorporalen Blutbehandlungsvorrichtung, die einen von einer semipermeablen Membran in eine erste Kammer und eine zweite Kammer unterteilten Dialysator oder Filter aufweist, wobei die erste Kammer des Dialysators oder Filters Teil eines extrakorporalen Blutkreislaufs und die zweite Kammer Teil eines Dialysierflüssigkeitssystems ist,
**dadurch gekennzeichnet, dass** die Vorrichtung (24) zur Auswahl eines Dialysators oder Filters aufweist:

eine Eingabeeinheit (24C) zum Eingeben von einem oder mehreren patientenspezifischen Behandlungsparametern, die für das Therapieziel und/oder den zu behandelnden Patienten charakteristisch sind,
eine Recheneinheit (24A), die derart ausgebildet ist,
dass ein maschinenspezifischer Behandlungsparameter aus dem einen oder mehreren patientenspezifischen Behandlungsparametern zur Durchführung der Behandlung unter Verwendung jeweils einer der Dialysatoren oder Filter aus einer Gruppe von unterschiedlichen Typen von Dialysatoren oder Filtern für sämtliche Typen von Dialysatoren oder Filtern bestimmbar ist und
dass die sich aus dem maschinenspezifischen Behandlungsparameter unter Verwendung des jeweiligen Dialysators oder Filters ergebenden Kosten der extrakorporalen Blutbehandlung für sämtliche Typen von Dialysatoren oder Filtern bestimmbar sind,

und eine Anzeigeeinheit (24D) zur Anzeige der sich aus dem maschinenspezifischen Behandlungsparameter unter Verwendung des jeweiligen Dialysators oder Filters ergebenden Kosten für sämtliche Typen von Dialysatoren oder Filter.

**8.** Vorrichtung nach Anspruch 7, **dadurch gekennzeichnet, dass** die Vorrichtung (24) eine Speichereinheit (24B) zum Speichern der Kosten der unterschiedlichen Typen von Dialysatoren oder Filter und der Kosten von Verbrauchsmaterialien und/oder der Energiekosten aufweist, und die Recheneinheit (24A) derart ausgebildet ist,
dass die Menge der jeweiligen Verbrauchsmaterialien und/oder die Menge an Energie berechnet wird, die bei der Durchführung der extrakorporalen Blutbehandlung mit dem bestimmten Betrag des maschinenspezifischen Behandlungsparameters verbraucht wird,
dass die Kosten der bei der extrakorporalen Blutbehandlung verbrauchten Verbrauchsmaterialien und/oder Energie aus den Kosten der Verbrauchsmaterialien und/oder Energiekosten einerseits und der berechneten Menge an Verbrauchsmaterialien und/oder Energie andererseits berechnet wird und
dass die Gesamtkosten für sämtliche Typen von Dialysatoren oder Filtern jeweils aus der Summe der gespeicherten Kosten des jeweiligen Dialysators oder Filters und den berechneten Kosten der bei der extrakorporalen Blutbehandlung verbrauchten Verbrauchsmaterialien und/oder Energie berechnet werden.

**9.** Vorrichtung nach Anspruch 7 oder 8, **dadurch gekennzeichnet, dass** die Anzeigeeinheit (24D) unterschiedliche Felder aufweist, in denen die unterschiedlichen Beträge des maschinenspezifischen Behandlungsparameters für die unterschiedlichen Dialysatoren oder Filter sowie die sich aus der Verwendung des jeweiligen Dialysators oder Filters und der Einstellung des jeweiligen maschinenspezifischen Behandlungsparameters ergebenden Gesamtkosten der extrakorporalen Blutbehandlung angezeigt werden.

**10.** Vorrichtung nach einem der Ansprüche 7 bis 9, **dadurch gekennzeichnet, dass** der zu bestimmende maschinenspezifische Behandlungsparameter der Dialysierflüssigkeitsfluss ist, mit dem Dialysierflüssigkeit durch die zweite Kammer des Dialysators oder Filters strömen soll.

**11.** Vorrichtung nach einem der Ansprüche 7 bis 10, **dadurch gekennzeichnet, dass** die Recheneinheit (24A) ausgebildet ist, dass derjenige Dialysator oder Filter ausgewählt wird, bei dem die sich für die Behandlung ergebenden Kosten am geringsten sind, und dass die Anzeigeeinheit (24D) derart ausgebildet ist, dass dieser Dialysator oder Filter angezeigt wird.

**12.** Vorrichtung nach einem der Ansprüche 7 bis 11, **dadurch gekennzeichnet, dass** ein patientenspezifischer Parameter ein Parameter aus der Gruppe von Parametern ist, die das Blutverteilungsvolumen V, die Dauer T der extrakorporalen Blutbehandlung, die Dialysedosis, die Clearance K oder den Blutfluss $Q_b$ im extrakorporalen Blutkreislauf I umfasst.

**13.** Extrakorporale Blutbehandlungsvorrichtung, die einen von einer semipermeablen Membran (2) in eine erste Kammer (3) und eine zweite Kammer (4) unterteilten Dialysator (1) oder Filter verfügt, wobei die erste Kammer (3) Teil eines extrakorporalen Blutkreislaufs (I) und die zweite Kammer (4) des Dialysators (1) oder Filters Teil eines Dialysierflüssigkeitssystems (II) ist, **dadurch gekennzeichnet, dass** die extrakorporale Blutbehandlungsvorrichtung eine Vorrichtung zur Auswahl eines Dialysators oder Filters nach einem der Ansprüche 7 bis 12 aufweist.

**14.** Computerprogrammprodukt, **dadurch gekennzeichnet, dass** die Verfahrensschritte des Verfahrens zur Auswahl eines Dialysators oder Filters nach einem der Ansprüche 1 bis 6 durchgeführt werden, wenn das Computerprogramm auf einer Datenverarbeitungseinrichtung läuft.

## Claims

**1.** A method for selecting a dialyser or filter from cost standpoints for an extracorporeal blood treatment with an extracorporeal blood treatment apparatus, which comprises a dialyser or filter divided by a semipermeable membrane into a first chamber and a second chamber, wherein the first chamber is part of an extracorporeal blood circuit and the second chamber of the dialyser or filter is part of a dialysing fluid system, **characterised by** the following process steps:

inputting one or more patient-specific treatment parameters on an input unit, which are characteristic of the therapeutic aim and/or the patient to be treated,
determining for all types of dialyser or filter, by means of a computing unit, a machine-specific treatment parameter from the one or from the plurality of patient-specific treatment parameters for performing the treatment using in each case one of the dialysers or filters from a group of different types of dialyser or filter,
determining for all types of dialyser or filter, by means of the computing unit, the cost of the extracorporeal blood treatment resulting from the ascertained machine-specific treatment parameter using the given dialyser or filter,
displaying the cost resulting from the machine-specific treatment parameter using the given dialyser or filter on a display unit for all types of dialyser or filter.

**2.** The method according to claim 1, **characterised in that** the step of determining the cost of the extracorporeal blood treatment comprises the following steps:

storing the costs of the various types of dialyser or filter in a memory unit,
storing the costs of consumables and/or the energy costs in the memory unit,
calculating in the computing unit the quantity of the respective consumables and/or the quantity of energy that is consumed in the performance of the extracorporeal blood treatment with the ascertained machine-specific treatment parameter,
calculating the costs of the consumables and/or energy consumed in the extracorporeal blood treatment from the costs of the consumables and/or energy costs on the one hand and the calculated quantity of consumables and/or energy on the other hand,
the total costs for all types of dialyser or filter being calculated with the computing unit in each case from the sum of the stored costs of the respective dialyser or filter and the calculated costs of the consumables and/or energy consumed in the extracorporeal blood treatment.

3. The method according to claim 1 or 2, **characterised in that** the different quantities of the machine-specific treatment parameter for the different dialysers or filters and the total cost of the extracorporeal blood treatment resulting from the use of the given dialyser or filter and the adjustment of the given machine-specific treatment parameter are displayed on the display unit.

4. The method according to any one of claims 1 to 3, **characterised in that** the machine-specific treatment parameter to be determined is the dialysing fluid flow with which dialysing fluid flows through the second chamber of the dialyser or filter.

5. The method according to any one of claims 1 to 4, **characterised in that** a patient-specific parameter is a parameter from the group of parameters which comprises blood distribution volume V, duration T of the extracorporeal blood treatment, the dialysis dose, clearance K or blood flow $Q_b$ in extracorporeal blood circuit I.

6. The method according to any one of claims 1 to 5, **characterised in that** the computing unit is used to select the dialyser or filter with which the cost resulting for the treatment is lowest, and **in that** this dialyser or filter is displayed on the display unit.

7. A device for selecting a dialyser or filter from cost standpoints for an extracorporeal blood treatment with an extracorporeal blood treatment apparatus, which comprises a dialyser or filter divided by a semipermeable membrane into a first chamber and a second chamber, wherein the first chamber of the dialyser or filter is part of an extracorporeal blood circuit and the second chamber is part of a dialysing fluid system,
**characterised in that** the device (24) for selecting a dialyser or filter comprises:

an input unit (24C) for inputting one or more patient-specific treatment parameters, which are characteristic of the therapeutic aim and/or the patient to be treated,
a computing unit (24A), which is designed in such a way

that a machine-specific treatment parameter can be determined, for all types of dialyser or filter, from the one or more patient-specific treatment parameters for performing the treatment using in each case one of the dialysers or filters from a group of different types of dialyser or filter and
that cost of the extracorporeal blood treatment resulting from the machine-specific treatment parameter using the given dialyser or filter can be determined for all types of dialyser or filter,

and a display unit (24D) for displaying, for all types of dialyser or filter, the cost resulting from the machine-specific treatment parameter using the given dialyser or filter.

8. The device according to claim 7, **characterised in that** the device (24) comprises a memory unit (24B) for storing the costs of the various types of dialyser or filter and the costs of consumables and/or the energy costs, and the computing unit (24A) is designed in such a way
that the quantity of the given consumables and/or the quantity of energy is calculated that is consumed in the performance of the extracorporeal blood treatment with a specific quantity of the machine-specific treatment parameter,
that the costs of the consumables and/or energy consumed in the extracorporeal blood treatment are calculated from the costs of the consumables and/or energy costs on the one hand and the calculated quantity of consumables and/or energy on the other hand, and
that the total costs for all types of dialyser or filter are calculated in each case from the sum of the stored costs of the respective dialyser or filter and the calculated costs of the consumables and/or energy consumed in the extracorporeal blood treatment.

9. The device according to claim 7 or 8, **characterised in that** the display unit (24D) comprises different fields, in which the different quantities of the machine-specific treatment parameter for the different dialysers or filters and the total cost of the extracorporeal blood treatment resulting from the use of the given dialyser or filter and the adjustment of the given machine-specific treatment parameter are displayed.

**10.** The device according to any one of claims 7 to 9, **characterised in that** the machine-specific treatment parameter to be determined is the dialysing fluid flow with which dialysing fluid is intended to flow through the second chamber of the dialyser or filter.

**11.** The device according to any one of claims 7 to 10, **characterised in that** the computing unit (24A) is designed in such a way that the dialyser or filter is selected with which the cost resulting for the treatment is lowest, and **in that** the display unit (24D) is designed in such a way that this dialyser or filter is displayed.

**12.** The device according to any one of claims 7 to 11, **characterised in that** a patient-specific parameter is a parameter from the group of parameters comprising blood distribution volume V, duration T of the extracorporeal blood treatment, the dialysis dose, clearance K or blood flow $Q_b$ in extracorporeal blood circuit I.

**13.** An extracorporeal blood treatment apparatus, which comprises a dialyser (1) or filter divided by a semipermeable membrane (2) into a first chamber (3) and a second chamber (4), wherein the first chamber (3) is part of an extracorporeal blood circuit (I) and the second chamber (4) of the dialyser (1) or filter is part of a dialysing fluid system (II), **characterised in that** the extracorporeal blood treatment apparatus comprises a device for selecting a dialyser or filter according to any one of claims 7 to 12.

**14.** A computer program product, **characterised in that** the process steps of the method for selecting a dialyser or filter are carried out according to any one of claims 1 to 6, when the computer program runs on a data processing device.

## Revendications

**1.** Procédé permettant de choisir un dialyseur ou un filtre, du point de vue des coûts, pour un traitement sanguin extracorporel avec un dispositif de traitement sanguin extracorporel qui présente un dialyseur ou un filtre divisé par une membrane semi-perméable en une première chambre et une deuxième chambre, la première chambre faisant partie d'une circulation sanguine extracorporelle et la deuxième chambre du dialyseur ou du filtre faisant partie d'un système de fluide de dialyse, **caractérisé par** les étapes suivantes du procédé :

entrée d'un ou plusieurs paramètres de traitement spécifiques d'un patient dans une unité d'entrée, qui sont caractéristiques de l'objectif thérapeutique et/ou des patients à traiter,
détermination d'un paramètre de traitement spécifique de la machine à partir d'un ou plusieurs paramètres de traitement spécifiques du patient pour la réalisation du traitement en utilisant respectivement l'un des dialyseur ou filtre d'un groupe de divers types de dialyseurs ou de filtres pour tous les types de dialyseurs ou filtres, avec une unité de calcul, détermination des coûts du traitement sanguin extracorporel découlant du paramètre de traitement spécifique de la machine déterminé en utilisant le dialyseur ou filtre respectif pour tous les types de dialyseurs ou filtres avec l'unité de calcul,
affichage des coûts découlant du paramètre de traitement spécifique de la machine en utilisant le dialyseur ou filtre respectif sur une unité d'affichage pour tous les types de dialyseurs ou filtres.

**2.** Procédé selon la revendication 1, **caractérisé en ce que** l'étape de détermination des coûts du traitement sanguin extracorporel comprend les étapes suivantes :

mémorisation des coûts des différents types de dialyseurs ou filtres dans une unité de mémoire,
mémorisation des coûts des matériaux consommables et/ou des coûts énergétiques dans l'unité de mémoire,
calcul de la quantité de matériaux consommables respectifs et/ou de la quantité d'énergie dans l'unité de calcul, qui est utilisée lors de la réalisation du traitement sanguin extracorporel avec le paramètre de traitement spécifique de la machine déterminé,
calcul des coûts des matériaux consommables et/ou énergétiques utilisés lors du traitement sanguin extracorporel à partir des coûts des matériaux consommables et/ou des coûts énergétiques d'une part et de la quantité calculée de matériaux consommables et/ou d'énergie d'autre part,
dans lequel avec l'unité de calcul, les coûts totaux pour tous les types de dialyseurs ou filtres sont calculés respectivement à partir de la somme des coûts mémorisés du dialyseur ou filtre respectif et des coûts calculés des matériaux consommables et/ou de l'énergie utilisés lors du traitement sanguin extracorporel.

**3.** Procédé selon la revendication 1 ou 2, **caractérisé en ce que** les montants différents du paramètre de traitement spécifiques de la machine pour les dialyseurs ou filtres différents et les coûts totaux du traitement sanguin extracorporel découlant de l'utilisation du dialyseur ou filtre respectif et de l'ajustement du paramètre de traitement spécifique de la machine respectif sont affichés sur l'unité d'affichage.

4. Procédé selon l'une des revendications 1 à 3, **caractérisé en ce que** le paramètre de traitement spécifique de la machine à déterminer est le flux de fluide de dialyse selon lequel le fluide de dialyse s'écoule dans la deuxième chambre du dialyseur ou filtre.

5. Procédé selon l'une des revendications 1 à 4, **caractérisé en ce qu'**un paramètre spécifique du patient est un paramètre du groupe des paramètres qui comprend le volume de répartition du sang V, la durée T du traitement sanguin extracorporel, la dose de dialyse, la clairance K ou le flux de sang $Q_b$ dans la circulation sanguine extracorporelle 1.

6. Procédé selon l'une des revendications 1 à 5, **caractérisé en ce qu'**avec l'unité de calcul, on choisit le dialyseur ou filtre avec lequel les coûts découlant du traitement sont les plus faibles et **en ce que** ce dialyseur ou filtre est affiché sur l'unité d'affichage.

7. Dispositif de sélection d'un dialyseur ou filtre du point de vue des coûts, pour un traitement sanguin extracorporel avec un dispositif de traitement sanguin extracorporel qui présente un dialyseur ou un filtre divisé par une membrane semi-perméable en une première chambre et une deuxième chambre, la première chambre du dialyseur ou filtre faisant partie d'une circulation sanguine extracorporelle et la deuxième chambre faisant partie d'un système de fluide de dialyse,
**caractérisé en ce que** le dispositif (24) pour la sélection d'un dialyseur ou filtre présente :

   une unité d'entrée (24C) pour entrer un ou plusieurs paramètres de traitement spécifiques d'un patient, qui sont caractéristiques de l'objectif thérapeutique et/ou des patients à traiter, une unité de calcul (24A) qui est conçue de telle sorte

      qu'un paramètre de traitement spécifique de la machine puisse être déterminé à partir d'un ou plusieurs paramètres de traitement spécifiques du patient pour la réalisation du traitement en utilisant respectivement l'un des dialyseur ou filtre d'un groupe de divers types de dialyseurs ou de filtres pour tous les types de dialyseurs ou filtres, et
      que les coûts du traitement sanguin extracorporel découlant du paramètre de traitement spécifiques de la machine en utilisant le dialyseur ou filtre respectif puissent être déterminés pour tous les types de dialyseurs ou filtres,

   et une unité d'affichage (24D) pour afficher les coûts résultants du paramètre de traitement spécifique de la machine en utilisant le dialyseur ou filtre respectif pour tous les types de dialyseurs ou filtres.

8. Dispositif selon la revendication 7, **caractérisé en ce que** le dispositif (24) présente une unité de mémoire (24B) pour mémoriser les coûts des différents types de dialyseurs ou filtres et les coûts des matériaux consommables et/ou les coûts énergétiques et l'unité de calcul (24A) est conçue de telle sorte
que la quantité de matériaux consommables respectifs et/ou la quantité d'énergie qui est utilisée lors de la réalisation du traitement sanguin extracorporel avec le niveau déterminé de paramètre de traitement spécifique de la machine soit calculée,
que les coûts des matériaux consommables et/ou énergétiques utilisés lors du traitement sanguin extracorporel soient calculés à partir des coûts des matériaux consommables et/ou des coûts énergétiques d'une part et de la quantité calculée de matériaux consommables et/ou d'énergie d'autre part, et
que les coûts totaux pour tous les types de dialyseurs ou filtres soient calculés respectivement à partir de la somme des coûts mémorisés du dialyseur ou filtre respectif et des coûts calculés des matériaux consommables et/ou de l'énergie utilisés lors du traitement sanguin extracorporel.

9. Dispositif selon la revendication 7 ou 8, **caractérisé en ce que** l'unité d'affichage (24D) présente des champs différents, dans lesquels les niveaux différents de paramètre de traitement spécifique de la machine pour les dialyseurs ou filtres différents et les coûts totaux du traitement sanguin extracorporelle découlant de l'utilisation du dialyseur ou filtre respectif et de l'ajustement du paramètre de traitement respectif spécifique de la machine sont affichés.

10. Dispositif selon l'une des revendications 7 à 9, **caractérisé en ce que** le paramètre de traitement spécifique de la machine à déterminer est le flux de fluide de dialyse selon lequel le fluide de dialyse peut s'écouler par la deuxième chambre du dialyseur ou filtre.

**11.** Dispositif selon l'une des revendications 7 à 10, **caractérisé en ce que** l'unité de calcul (24A) est conçue de telle sorte que l'on choisisse le dialyseur ou filtre avec lequel les coûts découlant du traitement sont les plus faibles et **en ce que** l'unité d'affichage (24D) est conçue de telle sorte que ce dialyseur ou filtre soit affiché.

**12.** Dispositif selon l'une des revendications 7 à 11, **caractérisé en ce qu'**un paramètre spécifique du patient est un paramètre du groupe des paramètres qui comprend le volume de répartition du sang V, la durée T du traitement sanguin extracorporel, la dose de dialyse, la clairance K ou le flux de sang $Q_b$ dans la circulation sanguine extracorporelle 1.

**13.** Dispositif de traitement sanguin extracorporel qui dispose d'un dialyseur (1) ou filtre divisé par une membrane semi-perméable (2) en une première chambre (3) et une deuxième chambre (4), la première chambre (3) faisant partie d'une circulation sanguine extracorporelle (I) et la deuxième chambre (4) du dialyseur (1) ou du filtre faisant partie d'un système de fluide de dialyse (II), **caractérisé en ce que** le dispositif de traitement sanguin extracorporel présente un dispositif de sélection d'un dialyseur ou filtre selon l'une des revendications 7 à 12.

**14.** Produit de programme informatique, **caractérisé en ce que** les étapes de procédé du procédé de sélection d'un dialyseur ou filtre sont réalisées selon l'une des revendications 1 à 6 lorsque le programme informatique est utilisé sur un dispositif de traitement des données.

1
2
3
3a
3b
4
4a
4b
5
6
7
8
9
10
11
12
13
14
15
16
17
18
19
20
23
24
24A
24B
24C
24D
24E
I
II

Fig. 1

24D
Kosten
Kt/V Routenplaner V6.3 K
Therapieziel
Kt/V
1,4
T
240
[min]
Methode
HD
HDF post
HDF prä
Patient
Qb
290
[ml/min]
V
35
[l]
Labordaten
HKT,TP,Rezirkulation
Details Therapie
Maschine
4008
5008
Flüsse
Flüsse
Filter
manuell
FX high flux
FX low flux
Ergebnis
Qb
Filter
Qd
sp Kt/V
b 2 m clear.
UFV [ml]
290
FX 100
500
1,44
70
2500
320
FX 100
400
1,47
66
2500
Speichern
Ansehen
Kosten
25
25A
25B
25C
25D
Fig. 2

25
24D
Kosten
QB 1
QB 2
FX 50
FX 60
FX 80
FX 100
QD
1000
900
600
500
Kt/V
1,36
1,41
1,41
1,44
Summe Kosten
21,2
20,57
17,89
17,26
Filterpreise
10,4
10,8
11,2
11,6
weitere Kosten
10,8
9,77
6,69
5,66
Details weitere Kosten
Permeat
1,69
1,54
1,07
0,92
Säure
2,92
2,64
1,79
1,51
Bicarbonat
5,3
4,79
3,25
2,74
Elektr. Strom
0,89
0,81
0,57
0,49

Fig. 3

25
24D
Kosten
QB 1
QB 2
FX 60
FX 80
FX 100
QD
600
500
400
Kt/V
1,42
1,45
1,47
Summe Kosten
17,49
16,86
16,23
Filterpreise
10,8
11,2
11,6
weitere Kosten
6,69
5,66
4,63
Details weitere Kosten
Permeat
1,07
0,92
0,77
Säure
1,79
1,51
1,23
Bicarbonat
3,25
2,74
2,23
Elektr. Strom
0,57
0,49
0,41

Fig. 4

24D
25
Kt/V Routenplaner V6.3 K
25A
Therapieziel
Kt/V
1,4
T
240
[min]
Methode
HD
HDF post
HDF prä
25B
Patient
Qb
290
[ml/min]
V
35
[l]
Labordaten
HKT,TP,Rezirkulation
25C
Details Therapie
Maschine
4008
5008
Flüsse
Flüsse
Filter
manuell
FX high flux
FX low flux
25D
Ergebnis
Qb
Filter
Qd
Qs
sp Kt/V
b 2 m clear.
UFV [ml]
290
FX 100
300
A Sub
1,41
88
2500
320
FX 80
300
A Sub
1,42
86
2500
Speichern
Ansehen
Kosten

Fig. 5

24D
25
Kosten
QB 1
QB 2
FX 60
FX 80
FX 100
QD
600
400
300
Kt/V
1,43
1,41
1,41
Summe Kosten
18,11
16,45
15,82
Filterpreise
10,8
11,2
11,6
weitere Kosten
7,31
5,25
4,22
Details weitere Kosten
Permeat
1,17
0,86
0,71
Säure
1,96
1,4
1,11
Bicarbonat
3,56
2,53
2,02
Elektr. Strom
0,62
0,46
0,38

Fig. 6

25
24D
Kosten
QB 1
QB 2
FX 60
FX 80
FX 100
QD
400
300
300
Kt/V
1,43
1,42
1,51
Summe Kosten
16,15
15,53
15,93
Filterpreise
10,8
11,2
11,6
weitere Kosten
5,35
4,33
4,33
Details weitere Kosten
Permeat
0,88
0,72
0,72
Säure
1,43
1,14
1,14
Bicarbonat
2,58
2,07
2,07
Elektr. Strom
0,47
0,39
0,39

Fig. 7

24D
Kt/V Routenplaner V 7.2
Therapieziel
Kt/V
1,4
T
240
[min]
Methode
HD
HDF post
HDF prä
Patient
Qb
300
[ml/min]
V
45
[l]
Labordaten
HKT,TP,Rezirkulation
Details Therapie
Maschine
4008
5008
Flüsse
Flüsse
Filter
FX high flux
FX high flux
Ergebnis
Qb
300
Filter
FX 100
Qd
800
Qs
A Sub
sp Kt/V
1,24
b 2 m clear.
107
Kosten [€]
22,48
Speichern
Ansehen
Kosten
25
25A
25B
25C
25D

Fig. 8

24D
25
25A
25B
25C
25D
Kt/V Routenplaner V 7.2
Therapieziel
Kt/V
1,4
T
240
[min]
Methode
HD
HDF post
HDF prä
Patient
Qb
300
[ml/min]
V
45
[l]
Labordaten
HKT,TP,Rezirkulation
Details Therapie
Maschine
4008
5008
Flüsse
Flüsse
Filter
FX high flux
FX high flux
Ergebnis
Qb
300
Filter
FX 100
Qd
600
Qs
A Sub
sp Kt/V
1,22
b 2 m clear.
102
Kosten [€]
21,3
Speichern
Ansehen
Kosten

Fig. 9

24D
25
25A
25B
25C
25D
Kt/V Routenplaner V 7.2
Therapieziel
Kt/V
1,4
T
240
[min]
Methode
HD
HDF post
HDF prä
Patient
Qb
350
[ml/min]
V
45
[l]
Labordaten
HKT,TP,Rezirkulation
Details Therapie
Maschine
4008
5008
Flüsse
Flüsse
Filter
FX high flux
FX high flux
Ergebnis
Qb
350
Filter
FX 100
Qd
600
Qs
A Sub
sp Kt/V
1,39
b 2 m clear.
111
Kosten [€]
21,42
Speichern
Ansehen
Kosten

Fig. 10

24D
25
25A
25B
25C
25D
Kt/V Routenplaner V 7.2
Therapieziel
Kt/V
1,4
T
255
[min]
Methode
HD
HDF post
HDF prä
Patient
Qb
320
[ml/min]
V
45
[l]
Labordaten
HKT,TP,Rezirkulation
Details Therapie
Maschine
4008
5008
Flüsse
Flüsse
Filter
FX high flux
FX high flux
Ergebnis
Qb
320
Filter
FX 100
Qd
700
Qs
A Sub
sp Kt/V
1,39
b 2 m clear.
109
Kosten [€]
22,22
Speichern
Ansehen
Kosten

Fig. 11

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1614438 A2
- WO 2007140993 A1